**Europäisches Patentamt**

**European Patent Office**

**Office européen des brevets**

(19)

(11) Veröffentlichungsnummer : **0 437 694 B1**

(12)
# EUROPÄISCHE PATENTSCHRIFT

(45) Veröffentlichungstag der Patentschrift :
**16.06.93 Patentblatt 93/24**

(51) Int. Cl.$^5$ : **A61K 31/20, A61K 31/23**

(21) Anmeldenummer : **90121385.0**

(22) Anmeldetag : **08.11.90**

(54) **Verwendung einer Hydroxyoctadecadiensäure, deren zur Ketoform oxidierte Säure sowie deren Derivate zur Therapie östrogenabhängiger Krankheiten, sowie pharmazeutische Zusammensetzungen, die diese enthalten.**

(30) Priorität : **16.01.90 DE 4001060**
**24.10.90 EP 90120434**

(43) Veröffentlichungstag der Anmeldung :
**24.07.91 Patentblatt 91/30**

(45) Bekanntmachung des Hinweises auf die
Patenterteilung :
**16.06.93 Patentblatt 93/24**

(84) Benannte Vertragsstaaten :
**AT BE CH DE ES FR GB IT LI NL SE**

(56) Entgegenhaltungen :
**EP-A- 0 097 059**
**EP-A- 0 201 159**

(56) Entgegenhaltungen :
**Patent Abstracts of Japan, Nr. 5 (C-467)(2852), 8. Januar 1988**
**Medical Hypotheses, Band 27, Nr. 4, Dezember 1986, S. 317-325, Longman Group UK Ltd; M.R. Buchanan et al.**

(73) Patentinhaber : **Boots Pharma GmbH**
**Oberer Weberberg 11**
**W-8884 Höchstädt/Donau (DE)**

(72) Erfinder : **Streber, August S.**
**Mühlweg 2**
**W-8909 Aichen (DE)**

(74) Vertreter : **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81 (DE)**

## Beschreibung

Diese Erfindung betrifft die Verwendung einer Hydroxyoctadecadiensäure, deren zur Ketoform oxidierte Säure oder deren Derivate zur Behandlung östrogenabhängiger Krankheiten, insbesondere zur Behandlung von Mammakarzinomen oder von benigner Prostatahyperplasie, sowie pharmazeutische Zubereitungen, die diese Stoffe enthalten.

Maligne oder benigne Geschwulste, deren Zellwachstum durch Sexualhormone gesteuert wird, sind beispielsweise Prostatatumore, benigne Prostatahyperplasie oder Mammakarzinome.

Die benigne Prostatahyperplasie kann durch eine Verminderung der Androgene, was mit einer relativen Erhöhung des Anteils der Östrogene im Hormonhaushalt verbunden ist, bedingt sein.

Aber nicht nur die Verminderung der Androgene, sondern auch eine gesteigerte Östrogenproduktion durch erhöhte Aromatase-Aktivität beim Manne wird heute als Ursache für die benigne Prostatahyperplasie angesehen. Besonders das reichhaltige Bindegewebe der Prostata spricht auf die Östrogene mit einem aktivierten Wachstum an.

Die benigne Prostatahyperplasie entsteht und wächst durch eine hormonelle Fehlsteuerung. Dabei wächst ein "Organ" und nicht nur eine Gewebeart, d.h. die benigne Prostatahyperplasie besteht aus Bindegewebe und Drüsenepithel, die irreguläre Knoten bilden, z.B. auch Drüsen ohne den zugehörigen Ausführungsgang. Nun benötigen beide Zellarten zum Wachstumsimpuls zwei verschiedene und gegensätzliche Hormone. Die Drüsenzellen antworten auf Androgene (z.B. Testosteron), das Bindegewebe dagegen reagiert auf Östrogene (z.B. Östradiol).

Als Symptome treten hierbei insbesondere vermehrter Harndrang, Störungen beim Wasserlassen, später Restharn und schliesslich akute Harnsperre auf, weil durch den raumgreifenden Prozess in der Umgebung der Harnröhre das Wasserlassen gedrosselt und verlegt wird. Es ist daher für die Therapie erforderlich, die durch die Geschwulst erschwerte Miktion eines Patienten zu verbessern, das Miktionsvolumen zu erhöhen und damit die Restharnmenge zu reduzieren oder zu beseitigen.

Die Aromatase spielt bei der normalen Biosynthese des weiblichen Sexualhormons, des Östradiols, eine wesentliche Rolle, da durch sie die Umwandlung des zuerst entstehenden Testosterons in das Östradiol (Aromatisierung) erfolgt. So beträgt die tägliche Testosteronausschüttung bei der Frau aufgrund dieses physiologischen Syntheseweges etwa 0,1 mg.

Die Aromatase ist ein Enzymsystem des Cytochrom P-450 mit einer NADPH-abhängigen Cytochrom-Reduktase. Nicht-steroidale Aromataseinhibitoren (z.B. Imidazole, Aminoglutethimid) wirken hauptsächlich hemmend durch reversible Komplexbildung mit einer Häm-Gruppe des Enzyms, während steroidale Hemmstoffe festbinden und das Enzymsystem auf Dauer ausschalten.

Durch Aromatase-Inhibitoren wird bei der Frau wie - auf niedrigerem Niveau - auch beim Manne die Umwandlung von Androgenen in Östrogene inhibiert, so dass eine Senkung des Östrogenspiegels erfolgt. Derartige Aromatasehemmer werden bisher zur Behandlung des Mammakarzinoms und in ersten Versuchen bei benigner Prostatahyperplasie eingesetzt. Da sie noch unverhältnismässig teuer sind und im Gegensatz zum Karzinom die benigne Prostatahyperplasie einen vergleichsweise geringen Leidenscharakter hat, sind sie auf dem Markt der Prostata-Präparate noch nicht vertreten.

Da die bisher verwendeten Mittel und Substanzen in sehr teueren Syntheseprozessen entstehen, besteht ein Bedürfnis nach gut wirkenden Substanzen der Aromatasehemmung, die für die Behandlung von Mammakarzinomen und Prostatahyperplasie erfolgreich verwendet werden können, dabei aber entweder in der Natur vorkommen oder verhältnismässig preiswert synthetisiert werden.

Der Erfindung lag daher die Aufgabe zugrunde, einen Stoff anzugeben, der aufgrund seiner Hemmwirkung erfolgreich zur Behandlung von Tumoren und anderen Krankheiten einsetzbar ist, die durch weibliche Sexualhormone gesteuert werden.

Diese Aufgabe wird erfindungsgemäss dadurch gelöst, dass eine Hydroxyoctadecadiensäure, deren zur Ketoform oxidierte Säure oder deren $C_1$ bis $C_4$-Alkylesterderivate, die bis zu drei Hydroxy- bzw. Oxogruppen im Molekül aufweist, wobei die Doppelbindungen und die Hydroxygruppe(n) bzw. die Oxogruppe(n) an den Kohlenstoffatomen der Positionen 9 bis 13 vorhanden sind, zur Herstellung eines Arzneimittels zur Behandlung östrogenabhängiger Krankheiten, insbesondere zur Behandlung von Mammakarzinomen oder benigner Prostatahyperplasie, verwendet werden.

Mit derartigen Säuren liegt somit erstmals eine Substanz vor, die sowohl eine anti-androgene Wirkung als auch eine anti-östrogene Wirkung in Form der Aromatasehemmung aufweist.

Erfindungsgemäss können bis zu drei Hydroxy- bzw. Oxogruppen pro Molekül vorhanden sein. Von diesen Verbindungen ist insbesondere die Trihydroxyoctadecadiensäure geeignet.

Erfindungsgemäss wurde gefunden, dass die eingangs genannte Hydroxyoctadecadiensäure oder deren zur Ketoform oxidierte Säure, die Oxooctadecadiensäure, die vorzugsweise in der Position 10 bzw. 11 eine trans-Konfiguration und in der Position 12 bzw. 9 eine cis-Konfiguration aufweist, sich insbesondere zur Herstellung von Arzneimitteln zur Behandlung von östrogenabhängigen Krankheiten eignet. Besonders geeignet sind daher die folgenden vier Säuren: 9-Hydroxy-10(trans)-12(cis)-octadecadiensäure, 9-oxo-10(trans)-12(cis)-octadecadiensäure, 13-Hydroxy-9(cis)-11(trans)-octadecadiensäure und 13-oxo-9(cis)-11(trans)-octadecadiensäure. Dabei hat sich überraschenderweise herausgestellt, dass die Ketoform der 9-Hydroxysäure eine im Vergleich zu dieser Hydroxyverbindung 10-fach bessere Wirkung bei der Behandlung von östrogenabhängigen Krankheiten aufweist.

Die genannten Fettsäuren sind bereits bekannt und werden teilweise auch schon in der Medizin verwendet.

So wird beispielsweise in der japanischen Offenlegungsschrift JP 62-164620-AI die Verwendung der 9-Hydroxy-10,12-octadecadiensäure als Antihypertensivum genannt, wie ungesättigten Fettsäuren ganz allgemein ein Antiarteriosklerose-Effekt zugeschrieben wird. In einer weiteren Veröffentlichung, der EP-A-0 097 059, werden topische Zusammensetzungen, die diese Hydroxyäuren enthalten, zur Hautbehandlung vorgeschlagen. Diese Zusammensetzungen stellen jedoch Kosmetikzusammensetzungen dar.

Aus Chemical Abstracts, Vol. 93 (1980), Nr. 202348a ist bekannt, dass 9-Hydroxy-10,12-octadecadiensäure ein (in-vitro-) Stoffwechselprodukt von Linolensäure in $VX_2$-Tumorgewebekultur ist. In diesem Zusammenhang ist der Stoffwechselmetabolismus dieser Fettsäure hinsichtlich seiner Rolle für den Knochenkalkspiegel diskutiert.

Aus Chemical Abstracts, Vol. 85 (1976), Nr. 173098w sind die 9-Hydroxy-10(trans)-12(cis)-octadecadiensäure und die 13-Hydroxy-9(cis)-11(trans)-octadecadiensäure sowie die zur Ketoform oxidierten Säuren dieser beiden genannten Säuren sowie zum Teil deren Methylester bekannt. Diese Säuren konnten aus Mitochondrien von Rinderherzen isoliert werden.

Aus Chemical Abstracts, Vol. 106 (1987), Nr. 138127k ist schliesslich die 9-Hydroxy-10(trans)-12(cis)-octadecadiensäure bekannt, die als Schutzsubstanz in Reispflanzen dient. In den beiden zuletzt genannten Druckschriften ist jedoch kein Hinweis auf die pharmakologischen Eigenschaften der dort beschriebenen Säuren gegeben. Insbesondere ist über eine pharmakologische Wirksamkeit der Oxooctadecadiensäuren nichts bekannt gewesen.

Anstelle der Säuren können auch die entsprechenden Esterderivate verwendet werden, da das im Körper vorhandene Enzym Esterase den Ester in die freie Säure umzuwandeln vermag. Als Esterderivate sind insbesondere die Niedrigalkylester mit 1 bis 4 Kohlenstoffatomen geeignet.

Die Hydroxyoctadecadiensäuren kommen in pflanzlichen und tierischen Geweben vor und können daraus isoliert werden. In Pflanzenextrakten der Brennesselwurzeln kommen die genannten Säuren sowohl isoliert als auch als Esterkomponente bei Glyceriden, Ceramiden und Phospholipiden vor. Diese genannten Hydroxyoctadecadiensäuren können aber auch halbsynthetisch aus Ölsäure und Linolsäure erzeugt werden. Aus technischer Linolsäure, die bis zu 30 % Ölsäure enthält, können beispielsweise diese Säuren relativ preisgünstig in guter Ausbeute hergestellt werden.

Nachfolgend wird die Herstellung der 9-Hydroxy-10(trans)-12(cis)-octadecadiensäure sowie der entsprechenden zur Ketoform oxidierten 9-Oxo-10(trans)-12(cis)-octadecadiensäure zur Erläuterung beschrieben. Die Herstellung der anderen Hydroxysäuren bzw. der entsprechenden Oxosäuren kann auf analoge Weise oder nach einem der bekannten Verfahren, die eingangs erwähnt wurden, erfolgen.

(1) HERSTELLUNG DER 9-HYDROXY-10(TRANS)-12(CIS)-OCTADECADIENSÄURE:

30 g Linolsäure (techn., Fluka, Neu-Ulm) werden in 1,8 l Phosphatpuffer, pH 5,5 (96 Teile 1/15 molare $KH_2PO_4$-Lösung und 4 Teile 1/15 molare $Na_2HPO_4$-Lösung) zusammen mit 20 ml Tween 20$^R$ (Emulgator) durch kräftiges Schütteln emulgiert. Diese Substratlösung gibt man nach nochmaligem Schütteln zu einer Suspension von 2,5 bis 3 kg homogenisierten Tomatenfrüchten (komplett) in ca. 7 l Phosphatpuffer, pH 5,5. Der pH-Wert der Mischung wird mit konzentrierter $Na_2HPO_4$-Lösung möglichst genau auf 5,3 bis 5,5 eingestellt und die Mischung wird 5 bis 7 Stunden unter Luftzutritt langsam gerührt. Danach wird vorsichtig mit 30 %-iger $H_2SO_4$ auf pH 2 bis 3 angesäuert und ca. 4 bis 5 l Ether werden zugefügt. Nach ca. 15-minütigem kräftigen Rühren der Gesamtmischung wird die gelartige orange Etherphase abdekantiert und durch Zentrifugation von Wasser separiert. Zu der gelartigen Etherphase werden weiterer Ether (ca. 1 l) und ca. 100 g $Na_2SO_4$ (wasserfrei) zugegeben und geschüttelt. Die klare Etherlösung wird vom ausgeflockten Rückstand abdekantiert, wobei letzterer noch dreimal mit Ether extrahiert wird. Die getrocknete, klare Etherphase wird im Vakuum bei 30°C eingeengt. Es bleiben das Roh-Hydroperoxid und nicht-umgesetztes Ausgangsmaterial als rot gefärbtes Öl (ca. 30 bis 40 g) zurück.

Die Mischung wird ohne Reinigung sofort in 150 ml 0,1 molarer Boraxlösung (pH 9) unter Zusatz von 150 ml MeOH suspendiert und zu der in Eis gekühlten Mischung werden 5 g Natriumborhydrid (Fluka) in Portionen zugegeben. Nach vollständiger Zugabe entfernt man die Kühlung und rührt 2 Stunden bei Raumtemperatur.

Nach vorsichtigem Ansäuern auf pH 2 bis 3 mit wässriger HCl wird die Mischung zweimal mit $CH_2Cl_2$ extrahiert und die $CH_2Cl_2$-Phase wird mit NaCl-Lösung gewaschen. Nach Entfernung von $CH_2Cl_2$ am Rotationsverdampfer erhält man die Zielverbindung als Rohprodukt (ca. 30 g).

Diese wird durch zweimalige Säulenchromatografie an je ca. 800 g Kieselgel mit Cyclohexan/Ethylacetat/Essigsäure (150:100:2 bzw. 250:100:2) gereinigt (DC-Kontrolle, Rf = 0,43 auf Polygram[R] Sil G/UV$_{254}$ - Machery & Nagel - mit Cyclohexan/EtOAc/AcOH 100:50:2).

Schliesslich werden ca. 7 bis 8 g der 9-Hydroxy-10(trans)-12(cis)-octadecadiensäure (D-Enantiomeres) als (bei Raumtemperatur) zähflüssiges gelbes Öl, welches auch bei -20°C nur begrenzt lagerfähig ist, erhalten.

(2) HERSTELLUNG DER 9-OXO-10(TRANS)-12(CIS)-OCTADECADIENSÄURE:

0,6 g der 9-Hydroxyoctadecadiensäure werden in 60 ml trockenem Methylenchlorid unter Rühren gelöst und 4 g $MnO_2$ (akt. nach Attenborough, Fluka - 90 %) zugegeben. Die Suspension wird im Dunkeln 15 Stunden bei Raumtemperatur gerührt und durch Celite® filtriert. Nach Entfernen des Lösungsmittels wird der Rückstand auf Kieselgelplatten mit Cyclohexan/EtOAc/MeOH (70:30:2) getrennt.

Der Rückstand aus der Elution der Bande mit Rf = 0,35 bis 0,40 wird je einmal aus n-Hexan und MeOH/$H_2O$ umkristallisiert und getrocknet. Ca. 0,1 g 9-Oxo-10(trans)-12(cis)-octadecadiensäure werden erhalten, welche ebenfalls wenig stabil ist.

Die Verabreichung des Wirkstoffes kann oral durch Tabletten, Kapseln oder Dragees oder durch Injektionen, insbesondere intramuskulär, erfolgen, wobei die Lösung der Wirksubstanz entweder eine ölige oder verdünnt-ethanolische Lösung darstellen kann.

Eine bevorzugte Arzneimittelzubereitung in Tablettenform enthält 9-Hydroxy-10(trans)-12(cis)-octadecadiensäure, 9-Oxo-10(trans)-12(cis)-octadecadiensäure, 13-Hydroxy-9(cis)-11(trans)-octadecadiensäure oder 13-Oxo-9(cis)-11(trans)-octadecadiensäure sowie weitere Zusatzstoffe, wobei vorzugsweise Lactose oder Maltodextrin sowie Kalzium-carboxymethylzellulose als Hilfsstoffe zugegeben werden.

Die Hemmversuche wurden mit einer Suspension von humanen Plazentamikrosomen (Aromatase) mit dem Substrat Androstendion im kompetitiven Hemmtest durchgeführt. Die Bedingungen sind in Tabelle 1 zusammengefasst.

TABELLE 1

Experimentelle Bedingung der Enzymhemmung

| | |
|---|---|
| Volumen des Testansatzes: | 500 /ul |
| Enzymquelle: | Plazentamikrosomen |
| | 40 /ug Protein |
| Substrat: | Androstendion (10 nM) |
| Tracer: | $^3$H |
| Co-Substrat: | NADPH-regenerierendes System |
| | (0,5 mM) |
| Inkubationszeit: | 10 Minuten |
| gemessenes Produkt: | $H_2O$ |
| Detektion: | LSC |

Die Aromataseaktivität der erfindungsgemäss verwendeten 9-Hydroxy-10(trans)-12(cis)-octadecadiensäure wurde mit der einer Kontrollgruppe ohne Hemmstoffzusatz verglichen.

Die Aromataseaktivität ist in Prozent Umsatz in Tabelle 2 angegeben.

TABELLE 2

| Aromataseaktivität[1] von 9-Hydroxy-10(trans)-12(cis)-octadecadiensäure | | | | | |
|---|---|---|---|---|---|
| ohne Hemmstoff | 100 ± 9,1 | | 100 ± 8,1 | | 100 ± 5,5 |
| | | Konzentration des Hemmstoffes | | | |
| | | 1 mg/l | 10 mg/l | | 100 mg/l |
| mit Hemmstoff | | 94,4 ± 1,2 | 53,8 ± 3,8 | | 16,3 ± 3,2 |

Die erhaltenen Werte belegen eine deutliche Hemmung der Aromatase durch die erfindungsgemäss verwendete Fettsäure 9-Hydroxy-10(trans)-12(cis)-octadecadiensäure.

[1] Angabe in pMol/10 Min/mg prot

Weitere Hemmversuche wurden mit 9-Oxo-10(trans)-12(cis)-octadecadiensäure und mit Trihydroxy-10(trans)-12(cis)-octadecadiensäure durchgeführt. Die Bedingungen der Hemmversuche sind in Tabelle 3 angegeben, die Aromataseaktivität ist in Prozent Umsatz in Tabelle 4 angegeben.

TABELLE 3

Experimentelle Bedingungen der Aromatasehemmung

Volumen des Testansatzes:      500 µl

Enzymquelle:                   Plazentamikrosomen
                               40 µg Protein

Substrat:                      Androstendion (100 nM)

Tracer:                        $^3$H

Co-Substrat:                   NADPH-regenerierendes System
                               (0,5 mM)

Inkubationszeit:               10 Minuten

gemessenes Produkt:            $H_2O$

Detektion:                     LSC

TABELLE 4

Aromataseaktivität[1] (% Umsatz)

| | Konzentration des Hemmstoffes | | |
| --- | --- | --- | --- |
| | 1 mg/l | 10 mg/l | 100 mg/l |
| ohne Hemmstoff | 100 ± 9,1 | 100 ± 8,1 | 100 ± 5,5 |
| mit Hemmstoff | | | |
| 1) 9-Oxo-10(trans)-12(cis)-octade-cadiensäure | 58,3 ± 3,6 | 19,3 ± 3,4 | 6,2 ± 2,9 |
| 2) Trihydroxy-10(trans)-12(cis)-octa-decadien-säure | 54,1 ± 7,6 | 31,3 ± 2,0 | 27,1 ± 5,4 |
| 1/2) Lösungs-mittel (Gegen-kontrolle) | 95,9 ± 8,6 | 89,6 ± 9,4 | 102,1 ± 7,2 |

Mittelwert ± SO (n = 3)

[1] Angabe in pMol/10 Min/mg prot

Die erhaltenen Werte belegen eine deutliche Hemmung der Aromatase durch die erfindungsgemäss verwendeten Fettsäuren

Das Lösungsmittel für die Fettsäuren wurde zur Gegenkontrolle ebenfalls untersucht.

Die erhaltenen Werte der absoluten Aromataseaktivität stellen Mittelwerte aus jeweils drei Messungen dar, zusätzlich ist noch die Standardabweichung angegeben; der Versuch ohne Hemmstoff stellt einen Mittelwert aus neun Messwerten dar.

Aus den Tabellen 2 und 4 ist die Aromataseaktivität der verwendeten Fettsäuren ersichtlich. Insbesondere ergibt sich, dass die Ketoform der Octadecadiensäure eine stärkere Aromatasehemmwirkung aufweist. Diese Wirkung ist etwa 10 mal stärker als bei der Säure in Hydroxyform, was sich in einer Verschiebung im Hinblick auf die Konzentration des Hemmstoffes zeigt. Die Trihydroxy-10(trans)-12(cis)-octadecadiensäure zeigt ebenfalls eine Hemmwirkung, deren Grössenordnung der Wirkung der oben genannten Oxosäure entspricht.

**Patentansprüche**

**Patentansprüche für folgende Vertragsstaaten : AT, BE, CH, DE, FR, GB, IT, LI, NL, SE**

1. Verwendung einer Hydroxyoctadecadiensäure, deren zur Ketoform oxidierte Säure oder der $C_1$ bis $C_4$-Alkylesterderivate der beiden Säuren, die bis zu drei Hydroxy- bzw. Oxogruppen im Molekül aufweist, wobei die Doppelbindungen und die Hydroxygruppe bzw. die Oxogruppe an den Kohlenstoffatomen der positionen 9 bis 13 vorhanden sind, zur Herstellung eines Arzneimittels zur Behandlung östrogenabhängiger Krankheiten, insbesondere zur Behandlung von Mammakarzinomen oder benigner Prostatahyperplasie.

2. Verwendung nach Anspruch 1, dadurch **gekennzeichnet,** dass die Hydroxyoctadecadiensäure ausgewählt wird aus 9-Hydroxy-10(trans)-12(cis)-octadecadiensäure oder 13-Hydroxy-9(cis)-11(trans)-octadecadiensäure.

3. Verwendung nach Anspruch 1, dadurch **gekennzeichnet,** dass die zur Ketoform oxidierte Säure ausgewählt wird aus 9-Oxo-10(trans)-12(cis)-octadecadiensäure oder 13-Oxo-9(cis)-11(trans)-octadecadiensäure.

4. Verwendung nach einem der Ansprüche 1 bis 3, dadurch **gekennzeichnet,** dass die Hydroxy- oder Oxooctadecadiensäure zur parenteralen Verabreichung in alkoholischer Lösung eingesetzt wird.

5. Verwendung nach einem der Ansprüche 1 bis 4, dadurch **gekennzeichnet,** dass das Arzneimittel 50 bis 100 mg der Hydroxyoctadecadiensäure oder deren zur Ketoform oxidierten Säure oder eine entsprechende Menge des Esterderivates enthält.

6. Pharmazeutische Zusammensetzung, dadurch **gekennzeichnet,** daß sie als Wirkstoff eine Hydroxyoctadecadiensäure oder deren zur Ketoform oxidierte Säure, ausgewählt aus 9-Hydroxy-10(trans)-12(cis)-octadecadiensäure oder 13-Hydroxy-9(cis)-11(trans)-octa-decadiensäure, 9-Oxo-10(trans)-12(cis)-octadecadiensäure oder 13-Oxo-9(cis)-11(trans)-octadecadiensäure, oder deren $C_1$-$C_4$-Alkylesterderivate sowie einen pharmazeutisch akzeptablen Träger und/oder Hilfsstoffe enthält.

**Patentansprüche für folgende Vertragsstaat : ES**

1. Verfahren zur Herstellung einer pharmazeutischen Zusammensetzung, umfassend eine Hydroxyoctadecadiensäure oder deren zur Ketoform oxidierte Säure, ausgewählt aus 9-Hydroxy-10(trans)-12-(cis)-octadecadiensäure, 13-Hydroxy-9(cis)-11(trans)-octadecadiensäure, 9-Oxo-10(trans)-12-(cis)-octadecadiensäure oder 13-oxo-9(cis)-11 (trans)-octadecadiensäure, oder deren $C_1$-$C_4$-Alkylesterderivate davon, als Wirkstoff, durch vermischen des Wirkstoffes in einer therapeutisch wirksamen Menge mit einem pharmazeutisch akzeptablen Träger und/oder Hilfsstoffen.

2. Verfahren nach Anspruch 1, dadurch **gekennzeichnet,** daß die Hydroxy- oder Oxooctadecadiensäure zur parenteralen Verabreichung in alkoholischer Lösung eingesetzt wird.

3. Verfahren nach Anspruch 1 oder 2, dadurch **gekennzeichnet,** daß 50 bis 100 mg der Hydroxyoctadecadiensäure oder deren zur Ketoform oxidierten Säure oder eine entsprechende Menge des Esterderivates als Wirkstoff verwendet wird.

4. Verfahren zur Herstellung einer pharmazeutischen Zusammensetzung nach einem der Ansprüche 1 bis 3, dadurch **gekennzeichnet,** daß sie zur Behandlung östrogenabhängiger Krankheiten, insbesondere zur Behandlung von Mammakarzinomen oder benigner Prostatahyperplasie, verwendet wird.

**Claims**

**Claims for the following Contracting States : AT, BE, CH, DE, FR, GB, IT, LI, NL, SE**

1. Use of a hydroxyoctadecadienoic acid, its acid oxidised to the keto form or the $C_1$ to $C_4$-alkyl ester derivatives of the two acids, which have up to three hydroxy or oxo groups in the molecule, the double bonds and the hydroxy group or the oxo group being present on the carbon atoms at positions 9 to 13, for producing a medicament for treating oestrogen-related illnesses, in particular for treating mammary carcinomas or benign prostatic hyperplasia.

2. Use according to claim 1, characterised in that the hydroxyoctadecadienoic acid is selected from 9-hydroxy-10 (trans)-12(cis)-octadecadienoic acid or 13-hydroxy-9 (cis)-11(trans)-octadecadienoic acid.

3. Use according to claim 1, characterised in that the acid oxidised to the keto form is selected from 9-oxo-10 (trans)-12(cis)-octadecadienoic acid or 13-oxo-9(cis)11(trans)-octadecadienoic acid.

4. Use according to one of claims 1 to 3, characterised in that the hydroxyoctadecadienoic acid or oxooctadecadienoic acid is used in alcoholic solution for parenteral administration.

5. Use according to one of claims 1 to 4, characterised in that the medicament contains 50 to 100 mg of the hydroxyoctadecadienoic acid or its acid oxidised to the keto form or a corresponding amount of the ester derivative.

6. Pharmaceutical composition, characterised in that it contains a hydroxyoctadecadienoic acid or its acid oxidised to the keto form, selected from 9-hydroxy-10 (trans)-12(cis)-octadecadienoic acid or 13-hydroxy-9(cis)-11(trans)-octadecadienoic acid, 9-oxo-10 (trans)-12(cis)-octadecadienoic acid or 13-oxo-9(cis)-11(trans)-octadecadienoic acid, or its $C_1$-$C_4$-alkyl ester derivatives as active ingredient, as well as a pharmaceutically acceptable excipient and/or auxiliaries.

**Claims for the following Contracting State : ES**

1. Process for preparing a pharmaceutical composition, comprising a hydroxyoctadecadienoic acid or its acid oxidised to the keto form, selected from 9-hydroxy-10 (trans)-12(cis)-octadecadienoic acid, 13-hydroxy-9 (cis)-11(trans)-octadecadienoic acid, 9-oxo-10(trans)-12(cis)-octadecadienoic acid or 13-oxo-9(cis)-11(trans)-octadecadienoic acid, or its $C_1$-$C_4$-alkyl ester derivatives thereof as active ingredient, by mixing the active ingredient in a therapeutically active amount with a pharmaceutically acceptable excipient and/or auxiliaries.

2. Process according to claim 1, characterised in that the hydroxyoctadecadienoic acid or oxooctadecadienoic acid is used in alcoholic solution for parenteral administration.

3. Process according to claim 1 or 2, characterised in that 50 to 100 mg of the hydroxyoctadecadienoic acid or its acid oxidised to the keto form, or a corresponding amount of the ester derivative is used as active ingredient.

4. Process for preparing a pharmaceutical composition according to one of claims 1 to 3, characterised in that it is used for treating oestrogen-related illnesses, in particular for treating mammary carcinomas or benign prostatic hyperplasia.

**Revendications**

**Revendications pour les Etats contractants suivants : AT, BE, CH, DE, FR, GB, IT, LI, NL, SE**

1. Utilisation d'un acide hydroxyoctadécadiénique, de l'acide oxydé sous la forme céto, ou des esters d'alkyle en $C_1$ à $C_4$ des deux acides, qui présente jusqu'à trois groupes hydroxy ou oxo dans sa molécule, les doubles liaisons et le groupe hydroxy ou le groupe oxo étant présents sur les atomes de carbone aux positions 9 à 13, pour la préparation d'un médicament pour le traitement de maladies dépendant des oestrogènes, en particulier pour le traitement des cancers du sein ou de l'hyperplasie prostatique bénigne.

2. Utilisation selon la revendication 1, caractérisée en ce que l'acide hydroxyoctadécadiénique est choisi parmi l'acide 9-hydroxy-10(trans)-12(cis)-octadécadiénique ou l'acide 13-hydroxy-9(cis)-11(trans)-octadécadiénique.

3. Utilisation selon la revendication 1, caractérisée en ce que l'acide oxydé sous la forme céto est choisi parmi l'acide 9-oxo-10(trans)-12(cis)-octadécadiénique ou l'acide 13-oxo-9(cis)-11(trans)-octadécadiénique.

4. Utilisation selon l'une des revendications 1 à 3, caractérisée en ce que l'acide hydroxy- ou oxooctadécadiénique est utilisé en solution alcoolique pour l'administration parentérale.

5. Utilisation selon l'une des revendications 1 à 4, caractérisée en ce que le médicament contient 50 à 100 mg de l'acide hydroxyoctadécadiénique ou de l'acide oxydé sous la forme céto, ou une quantité correspondante de l'ester.

6. Composition pharmaceutique, caractérisée en ce qu'elle contient comme substance active un acide hydroxyoctadécadiénique, ou l'acide oxydé sous la forme céto, choisi parmi l'acide 9-hydroxy-10(trans)-12(cis)-octadécadiénique ou l'acide 13-hydroxy-9(cis)-11(trans)-octadécadiénique, l'acide 9-oxo-10(trans)-12(cis)-octadécadiénique ou l'acide 13-oxo-9(cis)-11(trans)-octadécadiénique, ou leurs esters d'alkyle en $C_1$ à $C_4$, ainsi qu'un support et/ou un adjuvant pharmaceutiquement acceptables.

**Revendications pour l'Etat contractant suivant : ES**

1. Procédé de préparation d'une composition pharmaceutique comprenant un acide hydroxyoctadécadiénique, ou l'acide oxydé sous la forme céto, choisi parmi l'acide 9-hydroxy-10(trans)-12(cis)-octadécadiénique, l'acide 13-hydroxy-9(cis)-11(trans)-octadécadiénique, l'acide 9-oxo-10(trans)-12(cis)-octadécadiénique ou l'acide 13-oxo-9(cis)-11(trans)-octadécadiénique, ou leurs esters d'alkyle en $C_1$ à $C_4$, comme substance active, par mélange de la substance active dans une quantité thérapeutiquement efficace avec un support et/ou des adjuvants pharmaceutiquement acceptables.

2. Procédé selon la revendication 1, caractérisé en ce que l'acide hydroxy- ou oxooctadécadiénique est utilisé en solution alcoolique pour l'administration parentérale.

3. Procédé selon l'une des revendications 1 ou 2, caractérisé en ce qu'on utilise comme substance active 50 à 100 mg de l'acide hydroxyoctadécadiénique ou l'acide oxydé sous la forme céto, ou une quantité correspondante de l'ester.

4. Procédé de préparation d'une composition pharmaceutique selon l'une des revendications 1 à 3, caractérisé en ce que celle-ci est utilisée pour le traitement de maladies dépendant des oestrogènes, en particulier pour le traitement du cancer du sein ou de l'hyperplasie prostatique bénigne.